# EUROPÄISCHE PATENTANMELDUNG

(11) **EP 0 773 208 A1**
(43) Veröffentlichungstag der Anmeldung: **14.05.1997**
(21) Anmeldenummer: 96117830.8
(22) Anmeldetag: 07.11.1996
(51) Int. Cl.: C07C 53/50, C07C 51/58

(54) **Verfahren zur Herstellung von Perfluorpropionylfluorid**

(30) Priorität: 13.11.1995 DE 19542190
(71) Anmelder: HOECHST AKTIENGESELLSCHAFT, 65929 Frankfurt am Main (DE)
(72) Erfinder: Siegemund, Günter, Dr., 65719 Hofheim (DE); Franz, Raimund, Dr., 65779 Kelkheim (DE)

(57) **Zusammenfassung**

Die Erfindung betrifft ein Verfahren zur Herstellung von Perfluorpropionylfluorid (II) durch Isomerisierung von Hexafluorpropenoxid (I): 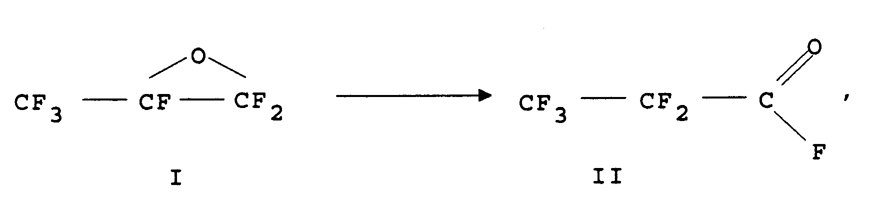 wobei man als Reaktionsmedium ein flüssiges, komplexes Ammonium-Hydrofluorid verwendet.

## Beschreibung

Die vorliegende Erfindung betrifft ein Verfahren zur selektiven Herstellung von Perfluorpropionylfluorid (II) durch Isomerisierung von Hexafluorpropenoxid (I): 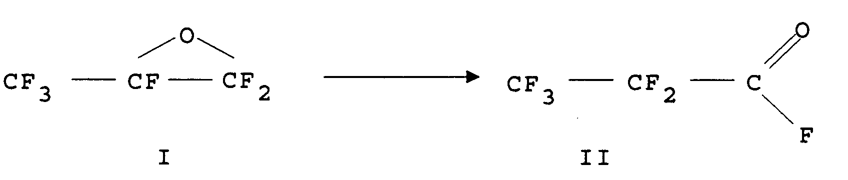 Hexafluorpropenoxid (I) und Perfluorpropionylfluorid (II) sind die Ausgangsstoffe für die Herstellung von Perfluor-(n-propyl-vinylether) PPVE, einem wertvollen Comonomer für die Produktion von modifiziertem Polytetrafluorethylen. Die Herstellung von PPVE verläuft gemäß dem folgenden Schema: 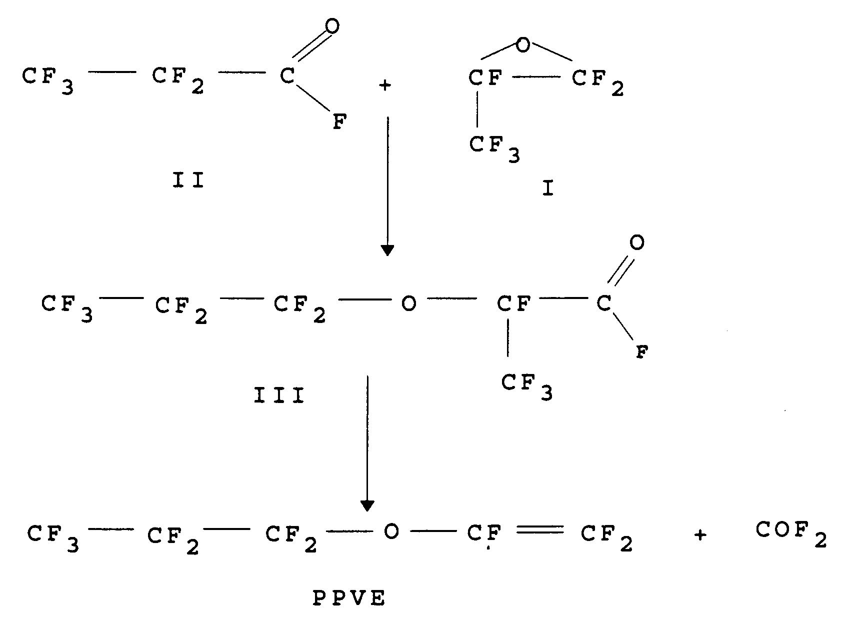 Hexafluorpropenoxid (I) kann also von seinem eigenen Isomerisierungsprodukt (II) unter Bildung eines Ether-Carbonsäurefluorids (III) angegriffen werden. Analog zur Reaktion von (II) mit (I) kann jedoch auch (III) mit (I) zu einem höheren Ether-Carbonsäurefluorid weiterreagieren, welches sich wiederum mit (I) umsetzt, so daß schließlich perfluorierte Polyether-Carbonsäurefluoride entstehen. Daher weisen die bekannten Verfahren der Isomerisierung von Hexafluorpropenoxid (I) zu Perfluorpropionylfluorid (II) erhebliche Nachteile hinsichtlich der Selektivität oder der Reaktionszeiten auf.

So wird zum Beispiel in der US-A-3 321 515 die Isomerisierung perfluorierter Epoxide beschrieben, die in Gegenwart katalytischer Mengen von Lewissäuren zu perfluorierten Ketonen, in Gegenwart katalytischer Mengen von Lewisbasen hingegen zu den entsprechenden Säurefluoriden führt. Als Lewissäuren werden saure Metalloxide und Metallhalogenide eingesetzt, als Lewisbasen hingegen Fluorionen in Form von Alkalimetallfluoriden oder Verbindungen, die im Reaktionsmedium Fluorionen erzeugen können, wie z. B. tertiäre Amine (inklusive Pyridin), deren N-Oxide, sowie tertiäre Säureamide. Eine Anzahl von Beispielen in der erwähnten Patentschrift beschreibt die Isomerisierung von Hexafluorpropenoxid (I) (HFPO) mit basischen Katalysatoren zu Perfluorpropionylfluorid (II). Dabei werden stets hohe Drücke angewandt oder sehr lange Reaktionszeiten oder beides. Die Reaktionen müssen daher grundsätzlich in Druckgefäßen durchgeführt werden, und häufig wird die Bildung eines öligen Nebenproduktes erwähnt. Direkt zugesetztes Fluorid ist als Katalysator offenbar wenig geeignet (Table II, Ex. Method 9 und 25), während mit (Fluoridionen erzeugenden) Aminen teilweise etwas bessere Ergebnisse erhalten werden (Ex. Method 16, 20, 21, 33, 36, 38, 56). Keiner der in der US-A-3 321 515 beschriebenen Katalysatoren bewirkt jedoch eine rasche und gleichzeitig drucklose Isomerisierung von Hexafluorpropenoxid (I) zu Perfluorpropionylfluorid (II) mit hohen Ausbeuten, wie sie für diese beiden leichtsiedenden Substanzen wünschenswert wäre.

Auch gemäß der viel später veröffentlichten japanischen Patentschrift 04 134 046 aus dem Jahr 1990, in der ebenfalls die Isomerisierung von Hexafluorpropenoxid (I) zu Perfluorpropionylfluorid (II) in Gegenwart von tert. Aminen, Pyridinen und Chinolinen beschrieben wird, erhält man das Carbonsäurefluorid nach 4 h Reaktionszeit nur mit einer Ausbeute von 61,9 % der Theorie. Es ist demnach offensichtlich sehr schwierig, das durch Isomerisierung gebildete Perfluorpropionylfluorid (II) an der Weiterreaktion mit Hexafluorpropenoxid (I) zu hindern. Diese Weiterreaktion wurde sogar bereits zur gezielten Herstellung von Gemischen perfluorierter Polyether-Carbonsäurefluoride ausgenutzt. So erhält man gemäß der DE-A-3 901 000 aus Hexafluorpropenoxid (I) in einem aprotischen Lösungsmittel in Gegenwart von Tetramethylethylendiamin ein Gemisch oligomerer Ether-Carbonsäurefluoride der allgemeinen Formel

C₂F₅-[CF₂OCF(CF₃)]ₙ-COF.

Vor dem Hintergrund dieses Standes der Technik war es nun sehr überraschend zu finden, daß Hexafluorpropenoxid (I) unter sehr milden Bedingungen selektiv zu Perfluorpropionylfluorid (II) isomerisiert wird, wenn man ein flüssiges, komplexes Ammonium-Hydrofluorid der allgemeinen Formel (III) als Reaktionsmedium verwendet

[R¹R²R³NH]⁺ [H₍ₙ₋₁₎Fₙ]⁻ III

Die Isomerisierung läuft dabei mit einer so hohen Geschwindigkeit ab, daß die Reaktion drucklos durchgeführt werden kann und die Verwendung druckfester Reaktoren unnötig wird. Jedoch schadet die Anwendung von Druck nicht.

Gegenstand der vorliegenden Erfindung ist daher ein Verfahren zur Herstellung von Perfluorpropionylfluorid (II) durch Isomerisierung von
Hexafluorpropenoxid (I) 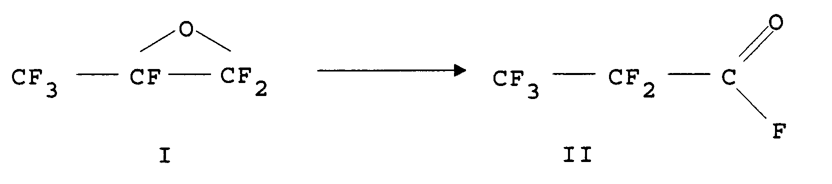 dadurch gekennzeichnet, daß man als Reaktionsmedium ein flüssiges, komplexes Ammonium-Hydrofluorid der allgemeinen Formel (III) verwendet,

[R¹R²R³NH]⁺ [H₍ₙ₋₁₎Fₙ]⁻ III

worin n eine ganze oder gebrochene Zahl ≤3 ist und
die Reste R¹, R² und R³ gleich oder verschieden sind und jeder dieser Reste
- einen: Alkylrest mit 1 bis 20 C-Atomen,
vorzugsweise 1 bis 12, insbesondere 1 bis 6 C-Atomen,
- einen: Cycloalkylrest mit 5 bis 7 C-Atomen,
- einen: Aralkylrest mit 7 bis 10 C-Atomen oder
- einen: Arylrest mit 6 bis 10 C-Atomen, der noch mit C₁- bis C₃-Alkyl- oder C₁ bis C₃-Alkoxygruppen substituiert sein kann,
bedeutet,
- oder: worin zwei der Reste R¹ bis R³ zusammen mit dem sie tragenden N-Atom einen 5- bis 7-gliedrigen Ring bilden, welcher ein O- oder ein weiteres N-Atom enthalten kann;
- oder: worin die Reste R¹ bis R³ zusammen mit dem sie tragenden N-Atom zwei oder drei 5- bis 7-gliedrige, gesättigte Ringe bilden, die weitere N-Atome enthalten können, wie z. B. im protonierten Diazabicyclooctan;
- oder: worin die Reste R¹ bis R³ zusammen einen 6-gliedrigen heterocyclischen Ring bilden, der ein oder zwei N-Atome enthalten und auch benzokondensiert sein kann, z.B. Pyridinium, Pyrimidinium oder Chinolinium.

Falls zwei der Reste R¹ bis R³ zusammen mit dem sie tragenden N-Atom einen 5- bis 7-gliedrigen Ring bilden, so enthält dieser Ring vorzugsweise kein O- und kein weiteres N-Atom.

Vorzugsweise ist mindestens einer der Reste R¹ bis R³ ein Alkylrest mit 1 bis 12 C-Atomen, insbesondere 1 bis 6 C-Atomen.

Besonders bevorzugt ist, daß alle drei Reste R¹ bis R³ Alkylreste sind und insgesamt 3 bis 12 C-Atome haben, insbesondere daß R¹ = R² = R³ = CH₃ ist.

Die Zahl n in der allgemeinen Formel (III) steht für eine ganze oder gebrochene Zahl ≤3, vorzugsweise 1,1 bis 2,9, insbesondere 2 bis 2,9.

Die Herstellung der komplexen Ammonium-Hydrofluoride kann durch direkte Umsetzung der Amine mit HF im gewünschten molaren Verhältnis erfolgen. Hierbei wird das Stickstoffatom des Amins protoniert; weitere HF-Moleküle vereinigen sich mit dem Fluorion unter Bildung komplexer Hydrofluorid-Anionen. Im folgenden werden einige Beispiele für die im erfindungsgemäßen Verfahren einsetzbaren, komplexen tert.-Ammonium-Hydrofluoride der allgemeinen Formel (III) angeführt:

(CH₃)₃NH⁺ H_{1,8}F_{2,8}⁻

(C₂H₅)₃NH⁺ H_{1,8}F_{2,8}⁻

(n-C₃H₇)₃NH⁺ H₂F₃⁻

(i-C₃H₇)₂(C₂H₅)NH⁺ H_{1,6}F_{2,6}⁻

(n-C₄H₉)₃NH⁺ H_{1,6}F_{2,6}⁻

[(CH₃)₂NH-CH₂-]₂²⁺ [H_{1,35}F_{2,35}]₂²⁻

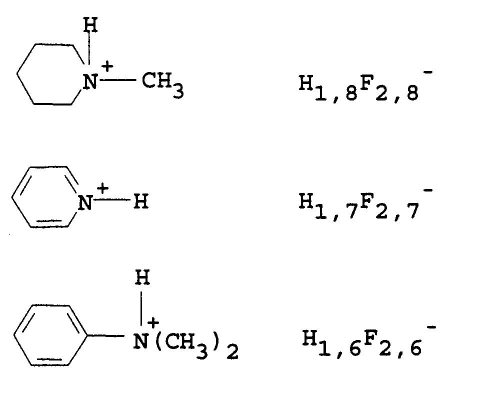

Komplexe Hydrofluoride dieser Art sind literaturbekannt, z. B. aus Bull. Soc. Chim. France 1965, Seite 1890 bis 1892, oder aus J. Fluorine Chemistry 15 (1980), Seite 423 bis 434. Es handelt sich hierbei um stabile Komplexe, die keinerlei Fluorwasserstoff-Dampfdruck aufweisen, daher einfach zu handhaben sind und teilweise sogar in Apparaturen aus Borsilikatglas destilliert werden können.

Das erfindungsgemäße Isomerisierungsverfahren kann unter wasserfreien Bedingungen in der für gasförmig/flüssige Reaktionsgemische üblichen Weise in Druckreaktoren, wie z. B. Rührautoklaven, durchgeführt werden. Jedoch ist die drucklose Durchführung in einer Blasensäule bevorzugt. Die Verwendung von Blasensäulen bei geeigneten Gas-Flüssigkeits-Umsetzungen ist Stand der Technik; die hierfür nötigen Anforderungen sind dem Fachmann bekannt.

Die Temperatur ist für den Isomerisierungsvorgang selbst nicht kritisch, sie richtet sich vielmehr nach den Eigenschaften des als Reaktionsmedium verwendeten komplexen tert.- Ammonium-Hydrofluorids (III), d. h. seinem Erstarrungspunkt, seiner temperaturabhängigen Viskosität bei Verwendung einer Blasensäule und seiner Zersetzungstemperatur. Die Isomerisierungstemperatur liegt im allgemeinen bei 0 bis 100°C, bevorzugt jedoch bei 40 bis 80°C.

### Beispiel 1

In ein als Blasensäule ausgerüstetes, von außen beheizbares Glasrohr von 2 m Länge und 25 mm Innendurchmesser, welches mit einem transparenten Tetrafluorethylen/Hexafluorpropen-Copolymer (FEP) ausgekleidet war, wurden als Medium 600 g des flüssigen, komplexen Ammonium-Hydrofluorids

[(CH₃)₃NH]⁺ [H_{1,8}F_{2,8}]⁻

bis zu einer Füllhöhe von 160 cm eingefüllt und auf 60°C erwärmt. Am Fuß der Blasensäule wurde sodann Hexafluorpropenoxid eingeleitet und in dem Medium fein verteilt. Das am Kopf der Blasensäule austretende Produktgas wurde in einer mit Trockeneis/Methanol gekühlten Falle kondensiert. Das Kondensat wurde ¹⁹F-NMR-spektroskopisch untersucht. Bezogen auf den Einsatz von Hexafluorpropenoxid wurde ein Umsatz von 95 Mol.-% ermittelt; bezogen auf diesen Umsatz betrug die Ausbeute an Perfluorpropionylfluorid 97,9 Mol.-%.

### Beispiel 2:

Die in Beispiel 1 beschriebene Umsetzung wurde wiederholt, jedoch bei einer Temperatur von nur 30°C. Aufgrund der nunmehr höheren Viskosität des Mediums wurde in der Blasensäule ein geringerer Durchsatz eingestellt als in Beispiel 1; die Verweilzeit des Gases im Medium war entsprechend höher. Gemäß NMR-Analyse des Produktgases betrug der Umsatz 95,9 Mol.-%, bezogen auf den Einsatz an Hexafluorpropenoxid; die auf diesen Umsatz bezogene Ausbeute an Perfluorpropionylfluorid hingegen war praktisch 100 Mol.-%.

### Beispiel 3:

Die in Beispiel 1 beschriebene Umsetzung wurde wiederholt, die Blasensäule dabei jedoch bei einer Temperatur von 80°C gehalten. Gemäß NMR-Analyse des Produktgases belief sich der Umsatz auf 88 Mol.-%, bezogen auf den Einsatz an Hexafluorpropenoxid; die auf diesen Umsatz bezogene Ausbeute an Perfluorpropionylfluorid betrug 98 Mol.-%.

### Beispiel 4:

In die in Beispiel 1 beschriebene Blasensäule wurden 520 g des flüssigen, komplexen Ammonium-Hydrofluorids

[(n-C₄H₉)₃NH]⁺ [H_{1,1}F_{2,1}]⁻

bis zu einer Höhe von 150 cm eingefüllt und auf 60°C erwärmt. Das Einleiten von Hexafluorpropenoxid und die Analyse wurden anschließend durchgeführt wie in Beispiel 1 beschrieben. Der Umsatz des eingesetzten Hexafluorpropenoxids war 100 Mol.-%; Perfluorpropionylfluorid war im Produktgas zu 96 Mol.-% enthalten.

### Beispiel 5:

Die in Beispiel 4 beschriebene Umsetzung wurde bei einer Temperatur von 70°C durchgeführt. Gemäß NMR-Analyse war der Umsatz des eingesetzten Hexafluorpropenoxids wiederum 100 Mol.-%; Perfluorpropionylfluorid war im Produktgas zu 99 Mol.-% enthalten.

### Beispiel 6:

In einem Rührautoklaven von 300 ml Fassungsvermögen (Werkstoff: ®Hastelloy C) wurden 100 g des flüssigen, komplexen Ammonium-Hydrofluorids

[(n-C₄H₉)₃NH]⁺ [H_{1,1}F_{2,1}]⁻

vorgelegt und aus einer Vorrats-Druckflasche etwa 20 g Hexafluorpropenoxid eingepreßt. Das Reaktionsgemisch wurde sodann 2 h unter Rühren auf 60°C unter Eigendruck erwärmt und anschließend über eine Kühlfalle im Trockeneisbad entspannt. Gemäß der ¹⁹F-NMR Analyse war in dem Gasgemisch Perfluorpropionylfluorid in einer Konzentration von 97,3 Mol.-% enthalten, neben 2,6 Mol.-% nicht umgesetztem Hexafluorpropenoxid. Weder im Kühlfalleninhalt, noch in dem im Autoklaven verbliebenen Hydrofluorid-Rückstand waren Dimerisierungs- oder Oligomerisierungsprodukte des Hexafluorpropens nachweisbar.

## Patentansprüche

1. Verfahren zur Herstellung von Perfluorpropionylfluorid (II) durch Isomerisierung von Hexafluorpropenoxid (I) 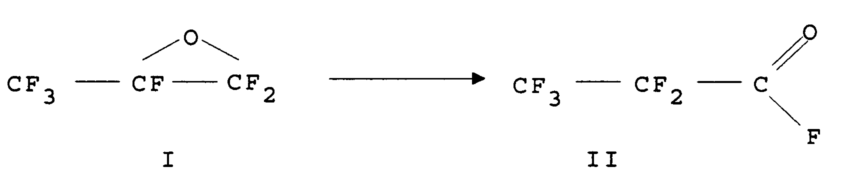 dadurch gekennzeichnet, daß man als Reaktionsmedium ein flüssiges,komplexes Ammonium-Hydrofluorid der allgemeinen Formel (III) verwendet,
[R¹R²R³NH]⁺ [H₍ₙ₋₁₎Fₙ]⁻ III
worin n eine ganze oder gebrochene Zahl ≤3 ist und
die Reste R¹, R² und R³ gleich oder verschieden sind und jeder dieser Reste
einen Alkylrest mit 1 bis 20 C-Atomen,
einen Cycloalkylrest mit 5 bis 7 C-Atomen,
einen Aralkylrest mit 7 bis 10 C-Atomen oder
einen Arylrest mit 6 bis 10 C-Atomen, der noch mit C₁- bis C₃-Alkyl- oder C₁ bis C₃-Alkoxygruppen substituiert sein kann,
bedeutet,
oder worin zwei der Reste R¹ bis R³ zusammen mit dem sie tragenden N-Atom einen 5- bis 7-gliedrigen Ring bilden, welcher ein O- oder ein weiteres N-Atom enthalten kann;
oder worin die Reste R¹ bis R³ zusammen mit dem sie tragenden N-Atom zwei oder drei 5- bis 7-gliedrige, gesättigte Ringe bilden, die weitere N-Atome enthalten können;
oder worin die Reste R¹ bis R³ zusammen einen 6-gliedrigen heterocyclischen Ring bilden, der ein oder zwei N-Atome enthalten und auch benzokondensiert sein kann.

2. Verfahren nach Anspruch 1, dadurch gekennzeichnet, daß man ein Ammonium-Hydrofluorid der Formel (III) einsetzt, bei dem mindestens einer der Reste R¹ bis R³ ein Alkylrest mit 1 bis 12 C-Atomen ist.

3. Verfahren nach Anspruch 1, dadurch gekennzeichnet, daß man ein Ammonium-Hydrofluorid der Formel (III) einsetzt, bei dem mindestens einer der Reste R¹ bis R³ ein Alkylrest mit 1 bis 6 C-Atomen ist.

4. Verfahren nach Anspruch 1, dadurch gekennzeichnet, daß man ein Ammonium-Hydrofluorid der Formel (III) einsetzt, bei dem alle drei Reste R¹ bis R³ Alkylreste sind und insgesamt 3 bis 12 C-Atome haben.

5. Verfahren nach Anspruch 1, dadurch gekennzeichnet, daß man ein Ammonium-Hydrofluorid der Formel (III) einsetzt, bei dem R¹ = R² = R³ = CH₃ ist.

6. Verfahren nach einem der Ansprüche 1 bis 5, dadurch gekennzeichnet, daß man ein Ammonium-Hydrofluorid der Formel (III) einsetzt, bei dem n eine ganze oder gebrochene Zahl von 1,1 bis 2,9 ist.

7. Verfahren nach einem der Ansprüche 1 bis 5, dadurch gekennzeichnet, daß man ein Ammonium-Hydrofluorid der Formel (III) einsetzt, bei dem n eine ganze oder gebrochene Zahl von 2 bis 2,9 ist.
